# EUROPEAN PATENT APPLICATION

(11) **EP 2 169 056 A1**
(43) Date of publication of application: **31.03.2010**
(21) Application number: 08765646.8
(22) Date of filing: 16.06.2008
(51) Int. Cl.: C12N 15/09, C12N 1/19, C12Q 1/02, C12Q 1/68

(54) **SET OF YEAST CELLS, METHOD OF IDENTIFYING TARGET CANDIDATE MOLECULE, METHOD OF ANALYZING ACTION MECHANISM AND SCREENING METHOD**

(30) Priority: 18.06.2007 JP 2007160457
(71) Applicant: Eisai R&D Management Co., Ltd., Tokyo 112-8088 (JP)
(72) Inventor: TSUKAHARA, Kappei, Tsukuba-shi Ibaraki 300-2635 (JP); MITSUHASHI, Kaoru, Tsukuba-shi Ibaraki 300-2635 (JP); IIDA, Makiyo, Tsukuba-shi Ibaraki 300-2635 (JP)
(74) Representative: Goddar, Heinz J.
(86) International application number: PCT/JP2008/060963
(87) International publication number: WO 2008/156048

(57) **Abstract**

Provided is a set of yeast gene knockout strains whose drug sensitivity is significantly improved. A set of yeast cells of the invention is a set of yeast cells comprising two or more types of yeast cells, wherein at least one mutually different gene has been deleted or mutated in each of the two or more types of yeast cells, and all of the two or more types of yeast cells are drug hypersensitive yeast cells in which the expression of at least one drug sensitivity-related gene is regulated.

## Description

### Technical Field

The present invention relates to a set of yeast cells, and a method of identifying a candidate target molecule, a method of analyzing a mechanism of action and a method of screening.

### Background Art

Although genome structures have become clear, identification of a mechanism by which a test substance acts on cells and of a target molecule of the test substance are still difficult issues. Based on the recent development of post-genome technologies such as DNA microarrays, the genome-wide analysis of actions of test substances has become possible (see, for example, Huels et al., 2002, Drug Discovery Today, 7 (suppl.): 119-124). A budding yeast *Saccharomyces cerevisiae* (*S. cerevisiae*) (hereinafter, sometimes referred to as "yeast"), which has many genes that are homologous to human genes and to which genetic techniques can be applied, has been regarded as a useful tool for clarifying a mechanism of action of a test substance (see, for example, Simon and Bedalov, Nat Rev Cancer, 2004, 4:481-92).

Recently, a project in which all of the about 6000 genes of yeast were disrupted one by one has been completed (see, for example, Giaever et al., Nature 2002 418:387-91), and a system using these yeasts has received much attention. The result of the above-described project revealed that about 1200 genes out of the about 6000 genes on the yeast genome are genes essential for survival (essential genes), and the knockout of any one of these genes is lethal to yeast. On the other hand, it was revealed that the remaining about 4800 genes are not essential to yeast (nonessential genes) and the yeast can grow in a normal culture medium, even when any one of these genes is disrupted.

If a test substance is interacted with each of the strains in which any one of the about 4800 nonessential genes are disrupted, and a nonessential-gene knockout strain specifically affected by the test substance is obtained, it is considered that the knocked-out gene relates to an action of the test substance. Here, the "nonessential gene" means a gene in which a cell can still grow even when the gene is disrupted (disruption of both of the two alleles in the case of a diploid cell, and disruption of one gene in the case of a haploid cell). Several methods for analyzing a mechanism of action of a test substance have been actually reported (see, for example, Giaever et al., Nat Genet, 1999, 21:278-83; Giaever et al., Proc Natl Acad Sci USA, 2004, 101:793-798; Lum et al., Cell, 2004, 116:121-37). Further, it has been reported that sites of action of test substances whose targets are unknown may be clarified by profiling test substance-sensitive strains for various test substances and comparing these profiles (see, for example, Parsons et al., Nat BioTechnol, 2004, 22:62-9).
Yeast strains used for the above-described purpose are generally called a yeast knockout strain (YKO), which are commercially available as a YKO collection.

Yeast cells have mechanisms for protecting themselves against a low-molecular-weight compound which is toxic to the yeast cells, since the yeast cells survive despite being exposed to various low-molecular-weight compounds in nature. For example, when a low-molecular-weight compound invades into a cell, the cell can protect against the low-molecular-weight compound by activating a pump that exports the compound to the outside of cell (hereinafter, sometimes referred to as a "transporter"). In a yeast cell, the composition of the plasma membrane is different from that of a mammalian cell. More specifically, the yeast cell contains ergosterol instead of cholesterol, and thus the plasma membrane permeability for low-molecular-weight compounds in yeast is much lower than that of the mammalian cell. It is known that, according to the development of such mechanisms, yeast cells are in general insusceptible to drugs (low-molecular-weight compounds) compared with mammalian cells. For example, in some drugs, the difference between the drug concentration at which it acts in yeast and that at which it acts in mammalian cells may be approximately 100 times.
Such low drug sensitivity of yeast cells has been a major impediment to the application of an evaluation method using YKO to various drugs.

As a method of enhancing drug sensitivity of yeast cells, a method for inhibiting expression of transcription factors Pdr1p and Pdr3p, which induce expression of ABC (ATP Binding Cassette) transporter, Pdr5p, has been reported (see, for example, Delaveau et al., Mol Gen Genet., 1994, 244(5), 501-11). Further, it is known that drug permeability can be enhanced by inhibiting the expression of an enzyme, Erg6p, which acts in the biosynthetic pathway of a structural component of the yeast cell membrane, ergosterol, thereby enhancing drug sensitivity of yeast cells (see, for example, International Patent Publication No. WO 2006/046694).

### Disclosure of Invention

An object of the present invention is to provide a set of yeast gene knockout strains whose drug sensitivity is significantly improved. Another object of the present invention is to provide a sensitive method of identifying a candidate target molecule for a test substance, a method of analyzing a mechanism of action of a test substance, and a method of screening a substance that affects a function of a target molecule, by using the set of yeast gene knockout strains whose drug sensitivity is significantly improved.

### Technical Solution

The present inventors found that the objects of the present invention can be attained by additionally regulating the expression of at least one drug sensitivity-related gene in each yeast cell included in a conventional set of yeast gene-knockout strains, thereby completing the present invention.
That is, specific methods of the present invention are as follows.

A first aspect of the present invention relates to a set of yeast cells, including two or more types of yeast cells, wherein at least one mutually different gene has been deleted or mutated in each of the two or more types of yeast cells, and all of the two or more types of yeast cells are drug hypersensitive yeast cells in which the expression of at least one drug sensitivity-related gene is regulated.
The at least one drug sensitivity-related gene is preferably at least one selected from a transporter-related gene or an ergosterol biosynthesis-related gene.
The transporter-related genes are preferably PDR1 and PDR3. The ergosterol biosynthesis-related gene is preferably ERG6 or ERG3.
The expression of the transporter-related gene is preferably regulated by deletion of the transporter-related gene, and the expression of the ergosterol biosynthesis-related gene is preferably regulated by transcriptional regulation of the ergosterol biosynthesis-related gene.
The yeast cells may be diploid.

A second aspect of the present invention relates to a method of identifying a candidate target molecule for a test substance, the method including the steps of:
contacting at least one type of yeast cells included in the above-described set of yeast cells with a test substance;
selecting a yeast cell whose phenotype was altered by contacting with the test substance;
identifying a gene mutated in the selected yeast cell as a first gene; and
identifying at least one of a transcription product or a translation product of the first gene as a candidate target molecule for the test substance.

A third aspect of the present invention relates to a method of identifying a candidate target molecule for a test substance, the method including the steps of:
contacting at least one type of yeast cells included in the above-described set of yeast cells with a test substance;
selecting a yeast cell whose phenotype was altered by contacting with the test substance;
identifying a gene deleted or mutated in the selected yeast cell as a first gene;
identifying a gene allelic to the first gene as a second gene; and
identifying at least one of a transcription product or a translation product of the second gene as a candidate target molecule for the test substance.

A fourth aspect of the present invention relates to method of identifying a candidate target molecule for a test substance, the method including the steps of:
contacting at least one type of yeast cells included in the above-described set of yeast cells with a test substance;
selecting a yeast cell whose phenotype was altered by contacting with the test substance;
identifying a gene deleted or mutated in the selected yeast cell as a first gene;
identifying a gene which is in a synthetic lethal relationship with the first gene as a second gene; and
identifying at least one of a transcription product or a translation product of the second gene as a candidate target molecule for the test substance.

A fifth aspect of the present invention relates to a method of analyzing a mechanism of action of a test substance with respect to yeast cells, the method including the steps of:
identifying a candidate target molecule for a test substance by the above-described method of identifying the candidate target molecule;
identifying a function of the candidate target molecule; and
analyzing a mechanism of action of the test substance with respect to yeast cells based on the function of the candidate target molecule.

A sixth aspect of the present invention relates to method of screening a substance that affects a function of a target molecule, the method including the steps of:
contacting yeast cells included in the above-described set of yeast cells with a test substance;
selecting a yeast cell whose phenotype was altered by contacting with the test substance;
identifying a gene mutated in the selected yeast cell as a first gene; and
selecting a test substance, where the first gene corresponds to a gene whose transcription product or translation product is the target molecule.

A seventh aspect of the present invention relates to a method of screening a substance that affects a function of a target molecule, the method including the steps of:
contacting yeast cells included in the above-described set of yeast cells with a test substance;
selecting a yeast cell whose phenotype was altered by contacting with the test substance;
identifying a gene deleted or mutated in the selected yeast cell as a first gene;
identifying a gene allelic to the first gene as a second gene; and
selecting a test substance, where the second gene corresponds to a gene whose transcription product or translation product is the target molecule.

An eighth aspect of the present invention relates to a method of screening a substance that affects a function of a target molecule, the method including the steps of:
contacting yeast cells included in the above-described set of yeast cells with a test substance;
selecting a yeast cell whose phenotype was altered by contacting with the test substance;
identifying a gene deleted or mutated in the selected yeast cell as a first gene; and
identifying a gene which is in a synthetic lethal relationship with the first gene as a second gene; and
selecting a test substance, where the second gene corresponds to a gene whose transcription product or translation product is the target molecule.

The alteration of the phenotype in the second to eighth aspects of the present invention is preferably lethality or growth inhibition.
The above-described step of selecting the yeast cell preferably includes the steps of:
(1) obtaining a function F(t) of a control curve of an index relating to the number of cells in the absence of a test substance over time, and a function G(t) of a test curve of an index relating to the number of cells in the presence of the test substance over time;
(2) calculating an integrated value of the formula (F(t)-G(t)) to obtain an area value; and
(3) selecting a yeast cell, whose phenotype was altered, based on the area value.

### Effect of Invention

According to the present invention, a set of yeast gene knockout strains whose drug sensitivity is significantly improved can be provided. The present invention also provides a sensitive method of identifying a candidate target molecule for a test substance, a method of analyzing a mechanism of action of a test substance, and a method of screening a substance that affects a function of a target molecule, by using the set of yeast gene knockout strains whose drug sensitivity is significantly improved.

### Brief Description of Drawings

Figure 1 is a schematic view showing a procedure for preparing MATa-type barrier-free YDS;
Figure 2 is a schematic view showing a procedure for preparing MATα-type barrier-free YDS;
Figure 3 is a schematic view showing a procedure for preparing MATa-type super barrier-free YDS;
Figure 4 is a graph showing the sensitivity of drug sensitivity-related gene knockout strains to lovastatin; and
Figure 5 is a graph showing the sensitivity of the drug sensitivity-related gene knockout strains to rapamycin.

### Best Mode of Carrying out the Invention

The set of yeast cells according to the present invention characteristically includes two or more types of yeast cells, wherein at least one mutually different gene has been deleted or mutated in each of the two or more types of yeast cells, and all of the two or more types of yeast cells are drug hypersensitive yeast cells in which the expression of at least one drug sensitivity-related gene is regulated.
The set of yeast cells with this configuration is a set of yeast gene knockout strains whose drug sensitivity is significantly improved.

The set of yeast cells according to the present invention includes two or more types of yeast cells. From the viewpoints of accuracy and efficiency of identification of the candidate target molecule in the below-described method of identifying a candidate target molecule for a test substance, the set preferably includes 1000 or more types of yeast cells, more preferably includes 2000 or more types of yeast cells, and still more preferably includes 3000 or more types of yeast cells.

In the two or more types of yeast cells of the present invention, at least one mutually different gene has been deleted or mutated in each of the two or more types of yeast cells. By using a set of yeast cells that include such yeast cells, a candidate target molecule for a test substance can be identified as described below.
The yeast cells in the present invention may be either haploid or diploid. When diploid yeast cells are used, one of the alleles of an essential gene is preferably deleted or mutated. In this case, a transcription product or a translation product of the essential gene can also be identified as a candidate target molecule. Further, in the case of a nonessential gene, it is also preferable that both of the alleles are deleted or mutated.

The set of yeast cells according to the present invention includes two or more types of yeast cells, in which at least one mutually different gene has been deleted or mutated in each of the two or more types of yeast cells, can be prepared by a method described, for example, in Science 2001, 294, 2364-2368. The set of yeast cells can also be obtained as #95401.H2 manufactured by Invitrogen or #YSC1066 manufactured by Open Biosystems.

All of the yeast cells included in the set of yeast cells according to the present invention are drug hypersensitive yeast cells, in which the expression of at least one drug sensitivity-related gene is regulated.
The drug sensitivity-related gene in the present invention may be any gene, as long as it can directly or indirectly regulate a drug sensitivity of yeast cells and suppression or overexpression of the gene does not cause lethality. Examples of the gene include genes involved in drug efflux from a cell, genes involved in drug-permeability of the cell membrane and genes involved in stress responses. More specifically, examples of the gene include a transporter-related gene, an ergosterol biosynthesis-related gene and a stress response-related gene.

The drug sensitivity-related gene(s) in the present invention are preferably at least one selected from a transporter-related gene and an ergosterol biosynthesis-related gene, and are preferably both a transporter-related gene and an ergosterol biosynthesis-related gene.
Further, two or more kinds of the same type of genes can be used as the drug sensitivity-related genes in the present invention.

The transporter in the present invention means a membrane protein located in the plasma membrane and involved in the transportation of a transport substrate through the plasma membrane. Examples of the transporter include an ABC transporter which uses ATP-hydrolysis energy for transportation and an SLC transporter which does not use ATP-hydrolysis energy for transportation. In the present invention, an ABC transporter is preferable.
Examples of the transporter-related gene in the present invention include a gene encoding a protein constituting a transporter, a gene encoding a transcription factor for a transporter gene, a gene encoding a protein which regulates an activity of a transporter, and a gene which regulates the expression of a transporter on the surface of the plasma membrane.
In the present invention, the transporter-related gene is preferably a gene related to the PDR5 gene, which encodes a type of ABC transporter, pdr5p. Among PDR5-related genes, it is preferable to use PDR1 and PDR3.

PDR1 and PDR3 herein mean genes encoding pdr1p and prd3p, respectively, which are master transcription factors for the PDR5 gene.
Examples of pdr1p and prd3p of yeast cell include those having known amino acid sequences, for example, designated by GenBank Accession No.CAA96713 and GenBank Accession No. CAA84822 when the yeast is *Saccharomyces cerevisiae.*

Examples of the ergosterol biosynthesis-related gene include a gene encoding an ergosterol synthase protein, a gene encoding a transcription factor for an ergosterol synthase, and a gene which regulates an activity of an ergosterol synthase.
In the present invention, the ergosterol biosynthesis-related gene is preferably a gene encoding an ergosterol synthase protein. The gene encoding an ergosterol synthase protein is preferably ERG6 or ERG3, more preferably ERG6, when the yeast is *Saccharomyces cerevisiae.* Here, ERG6 and ERG3 are genes encoding erg6p and erg3p, respectively, each of which is an ergosterol synthase. Examples of erg6p and erg3p in the present invention include those having known amino acid sequences designated by GenBank Accession Nos. CAA89944 and CAA97586.

Examples of the stress response-related gene include YAP1 (GenBank Accession No.CAA89945).

The expression "the expression of at least one drug sensitivity-related gene is regulated" means that the drug sensitivity-related gene is partially or entirely deleted, or that expression of the drug sensitivity-related gene is regulated by transcriptional regulation.
The expression "drug sensitivity-related gene is partially deleted" means that the drug sensitivity-related gene lacks a part of its gene sequence to such an extent that the protein encoded by the gene cannot fulfill its function. The expression "cannot fulfill its function" may mean a state in which the function is suppressed to such an extent that sensitivity to a drug increases.

In the present invention, regulation of the expression of a drug sensitivity-related gene by the transcriptional regulation is preferably inducible suppression of the expression of the drug sensitivity-related gene. The expression "inducible suppression of the expression of the drug sensitivity-related gene" means that an expression-suppressed state and an expression-induced state can be controlled by an external factor. The "expression-suppressed state" may be a state in which the expression of a drug sensitivity-related gene is suppressed to such an extent that drug sensitivity increases, and preferably a state in which the expression level of a drug sensitivity-related gene is decreased, for example, to 10% or less of that of the wild-type strain or an unmodified strain. The "expression-induced state" is preferably a state in which the expression of the drug sensitivity-related gene is induced up to approximately the same level as that of the wild-type strain or an unmodified strain, and more preferably a state in which the expression is induced to 80% or more of that of the wild-type strain or an unmodified strain. Expression level of a drug sensitivity-related gene can be confirmed by, for example, using known techniques such as a Northern blotting or an RT-PCR technique.

Examples of a method for regulating the expression level of the drug sensitivity-related gene include a method of regulating transcription of the drug sensitivity-related gene. As the method of regulating transcription of the drug sensitivity-related gene, a known method can be used without limitation. More specifically, examples thereof include a method using a tetracycline-inducible gene expression system and a method using an inducible promoter.
Examples of the method of regulating transcription of the drug sensitivity-related gene that can be used in the present invention include those described in International Patent Publication No. WO 2006/046694.

In the present invention, from the viewpoints of drug sensitivity and types of drug-hypersensitive yeast strains which can be prepared, the regulation of expression of the drug sensitivity-related gene is preferably regulation of the gene expression by deletion of the transporter-related gene and by transcriptional regulation of the ergosterol biosynthesis-related gene.

In the present invention, when transcription of a drug sensitivity-related gene is regulated by an inducible promoter, it is preferable that, in addition to the drug sensitivity-related gene, the expression of a marker for selecting transformed yeast strains is also regulated by the inducible promoter. In this way, the effect of the marker gene can be effectively eliminated in a bioassay using the set of yeast cells of the present invention.
As the marker gene, a known marker gene such as KanMX6, MPR1 or PPR1+ can be used without limitation. In the present invention, from the viewpoint of selection efficiency, the marker is preferably PPR1+.

Specifically, the drug-hypersensitive yeast cell in the present invention is more preferably a yeast cell in which both of the PDR1 gene and PDR3 are deleted and the expression of the ERG6 gene is inducibly suppressed. In this way, the yeast having higher drug sensitivity can be obtained. Further, based on the improved mating efficiency, many drug-hypersensitive yeast cell lines can be obtained.

When the set of yeast cells of the present invention is composed of haploid yeast cells, the sex (mating type) of the yeast cells is α-mating type or α-mating type.
A set of yeast cells composed of yeast cells of α-mating type can be prepared by, for example, a method described in Science, 2001, 294, 2364-2368. On the other hand, a set of yeast cells composed of yeast cells of α-mating type can be prepared by, for example, carrying out counter-selection with 5-FOA (5-Fluoroorotic acid) using the URA3 gene as the marker gene, in the preparation of the set of yeast cells composed of yeast cells having the MATa gene. That is, since 5-FOA is converted to a toxic compound by the gene product of the URA3 gene, only the cells expressing the URA3 gene can be selectively eliminated by addition of 5-FOA to the culture medium.
In the selection of haploid yeast cells of the present invention, selection by a selection medium is preferably carried out at least twice. In particular, it is preferable to perform a single-colony selection at least once. By this process, contamination with diploid yeast cells having the wild-type gene can be effectively prevented.

In the present invention, from the viewpoint of preparation efficiency of diploid yeast cells, the URA3 gene is preferably used as the marker gene. For example, introduction of the URA3 gene whose expression is regulated by an MFA1 promoter allows the URA3 gene to express specifically in α-mating type yeast cells, whereby the α-mating type yeast cells can be selected using a uracil-free (Ura⁻) culture medium and α-mating type yeast cells can be selected using a culture medium containing 5-FOA. That is, a set of α-mating type yeast cells can be prepared simultaneously in the step of preparing a set of yeast cells of α-mating type.
Furthermore, by using an MFα1 promoter in place of the MFA1 promoter, a set of α-mating type yeast cells can be prepared in the same manner as in the preparation process of the set of α-mating type yeast cells.

The set of yeast cells of the present invention can be prepared by, for example, preparing a partner strain in which the expression of at least one type of drug sensitivity-related gene is regulated, and mating the partner strain with each of yeast cell lines included in a known set of yeast gene knockout strains (YKO). In the present invention, since the expression of the ERG6 gene is inducibly regulated, drug-hypersensitive yeast cells can be prepared more efficiently.
The partner strain in which the expression of at least one type of drug sensitivity-related gene is regulated can be prepared by, for example, using the method for efficient preparation of gene knockout alleles by PCR (Baudin A et al., Nucl.Acid Res., 1993, 21, 3329-3330), as a partner strain in which at least one type of drug sensitivity-related gene is disrupted.

Further, a partner strain in which the expression of at least one type of drug sensitivity-related gene is inducibly regulated can be prepared by, for example, using the method described in International Patent Publication No. WO 2006/046694.
Further, a yeast strain, in which the expression of at least one type of drug sensitivity-related gene is regulated and which further has another mutation, can be prepared, for example, according to the method described in Science, 2001 Dec 14; 294(5550): 2364-2368 (a paper on the Synthetic Genetic Array (SGA) method by Boone et al. of the University of Toronto).

A diploid yeast cell can be prepared by crossing (mating) of haploid cells.
For example, a homozygous diploid drug-hypersensitive yeast cell in which both alleles of a nonessential gene are deleted or mutated can be obtained by simultaneously preparing the α-mating type yeast cells and α-mating type yeast cells, both of which lack the nonessential gene, by using the URA3 gene as the marker as described above, and mating the α-mating type yeast cells and α-mating type yeast cells.
A heterozygous diploid drug-hypersensitive yeast cell in which one of the alleles of an essential gene is deleted or mutated can be prepared, for example, as follows. The deleted or mutated essential gene is prepared as a plasmid and introduced to a heterozygous diploid yeast cell line which is not drug-hypersensitive, and then allowed to sporulate. A haploid yeast cell line lacking the essential gene and having the plasmid is then selected from obtained spores. The selected haploid yeast cell line is mated with a partner strain, in which at least one drug sensitivity-related genes is disrupted, and then allowed to sporulate. Subsequently, for example, an α-mating type yeast cell line is selected. The diploid drug-hypersensitive yeast cell in which one of the alleles of an essential gene is deleted or mutated can be obtained by mating the selected yeast cell line again with the partner strain to obtain a diploid cell line, and then removing the introduced plasmid.

Regarding the specific working method in the above preparation method, for example, the method described in Molecular Cloning: A Laboratory Manual, Cold Spring Harbor, NY; or Methods in Yeast Genetics: A Cold Spring Harber Laboratory Course Manual, 2000 Edition, can be used.

The first method of identifying a candidate target molecule for a test substance according to the present invention characteristically includes the steps of:
contacting yeast cells included in the above-described set of yeast cells with a test substance;
selecting a yeast cell whose phenotype was altered by contacting with the test substance;
identifying a gene mutated in the selected yeast cell as a first gene; and
identifying at least one of a transcription product or a translation product of the first gene as a candidate target molecule for the test substance.
Since the first method of identifying a candidate target molecule for a test substance according to the present invention has the above steps, the candidate target molecule for the test substance can be identified with a higher degree of accuracy even when using a low concentration of a drug, at which concentration the drug could not be identified by a conventional method.

In the present invention, at least one type of yeast cells selected from the above-described set of yeast cells is contacted with a test substance. The number of the types of yeast cells is not particularly limited. From the viewpoints of accuracy and efficiency of identification of the candidate target molecule for the test substance, the number of the types of yeast cells is preferably as high as possible. For example, the candidate target molecule for the test substance can be identified efficiently and with a higher degree of accuracy by using preferably 1000 types or more of yeast cells, and more preferably 3000 types or more of yeast cells.

Any substance may be used as the test substance, as long as the substance can be brought into contact with yeast cells. Examples of the test substance include naturally-occurring low-molecular-weight compounds, naturally-occurring high-molecular-weight compounds, synthetic low-molecular-weight compounds, synthetic high-molecular-weight compounds, peptides, proteins, polysaccharides and nucleic acids.
The method of contacting the yeast cells with the test substance is not particularly limited, and examples thereof include a method in which a solution of the test substance is added to a culture medium of the yeast cells.

The change of phenotype may be a change of any phenotype as long as it can be identified, and examples thereof include a change of the number of cells, a change of colony size, a change of morphology and a change of color. From the viewpoint of detection efficiency, the change of phenotype is preferably lethality or growth inhibition.
Examples of the method of selecting a yeast cell whose phenotype has been changed to result in lethality or growth inhibition include a method in which the selection is conducted by evaluating the ratio of the cell number in the presence of the test substance to the cell number in the absence of the test substance in a culture medium of the yeast cells. This evaluation may be performed by actually counting the number of cells in the culture medium, or may be performed by setting an index relating to the number of cells and quantifying the index. Examples of the index relating to the number of cells include glucose consumption, the turbidity of the culture medium, the area of the cells, and fluorescence intensity. In the present invention, from the viewpoints of accuracy and simplicity, the index is preferably the turbidity of the culture medium. The turbidity of the culture medium can be obtained by, for example, measuring the light absorbance thereof at between 600 nm and 660 nm.

Examples of the method of quantifying the index relating to the number of cells include a method in which the index after culturing the cells for a certain period is measured, and a method in which the changing rate of the index is evaluated by measuring the index over time.

In the present invention, the step of a selecting the yeast cell preferably includes the steps of:
(1) obtaining a function F(t) of a control curve of an index relating to the number of cells in the absence of a test substance over time, and a function G(t) of a test curve of an index relating to the number of cells in the presence of the test substance over time;
(2) calculating an integrated value of the formula (F(t)-G(t)) to obtain an area value; and
(3) selecting a yeast cell, whose phenotype was altered, based on the area value.
   By this method, the yeast cell whose phenotype was changed can be selected with a higher degree of accuracy and with excellent reproducibility.

In the present invention, from the viewpoints of accuracy and reproducibility, the step (2) to obtain the area value is preferably a step in which the area value is obtained by calculating an integrated value of the formula (F(t)-G(t)) within a predetermined time period, and is more preferably a step in which the area value is obtained by dividing an integrated value of the formula (F(t)-G(t)) within a predetermined time period by the length of the predetermined time.

In the present invention, the function F(t) can be obtained by, for example, measuring the index relating to the number of cells over time and then approximating the change of the index relating to the number of cells over time by a straight line. The function G(t) can also be obtained in the same manner as the function F(t).
For the step for obtaining the area value, for example, the description in Japanese Patent Application Laid-Open No. 2006-141298 can also be applied appropriately to the present invention.

In the step of identifying a gene deleted or mutated in the selected yeast cells as a first gene, the gene deleted or mutated can be identified by analyzing the gene in the selected yeast cell using a known method such as PCR. Further, the gene deleted or mutated in the selected yeast cell can be easily identified by using, as the yeast cell of the present invention, a yeast cell line in which a gene deleted or mutated has already been identified.

For example, in the step of identifying at least one of a transcription product or a translation product of the first gene as the candidate target molecule for the test substance, when the mutation of the first gene is a mutation that reduces a function of the transcription product or the translation product, at least one of the transcription product or the translation product of the first gene can be identified as the candidate target molecule for the test substance.

Examples of the yeast cell line having a mutation that reduces a function of a transcription product or a translation product include a temperature-sensitive yeast cell line caused by a mutation of the first gene and a yeast cell line in which the expression of the first gene is incompletely suppressed.

The second and third methods of identifying a candidate target molecule for a test substance according to the present invention characteristically include the steps of:
contacting yeast cells included in the above-described set of yeast cells with a test substance;
selecting a yeast cell whose phenotype was altered by contacting with the test substance;
identifying a gene deleted or mutated in the selected yeast cell as a first gene;
identifying a gene which is allelic to the first gene or a gene which is in a synthetic lethal relationship with the first gene as a second gene; and
identifying at least one of a transcription product or a translation product of the second gene as a candidate target molecule for the test substance.

Since the second and third methods of identifying a candidate target molecule for a test substance according to the present invention have the above steps, the candidate target molecule for the test substance can be identified with a higher degree of accuracy even when using a low concentration of a drug, at which concentration the drug could not be identified using a conventional method.

In the second and third methods of identifying a candidate target molecule for a test substance according to the present invention, the steps in common with the first method may be explained by the description of the corresponding steps of the first method of identifying a candidate target molecule.

For example, in the step of identifying a gene allelic to the first gene as a second gene, when the first gene is an essential gene and the yeast cell line is diploid, a gene allelic to the first gene can be identified as a second gene.

For example, in the step of identifying a gene which is in a synthetic lethal relationship with the first gene as a second gene, when the first gene is a nonessential gene and the yeast cell line is haploid or diploid, a gene which is in a synthetic lethal relationship with the first gene can be identified as a second gene.

Here, "synthetic lethal" means a property that causes lethality when plural genes in a cell are disrupted simultaneously. When a combination of genes causes synthetic lethality, the genes are in a synthetic lethal relationship. "Allelic genes" indicate a relationship, in the case of a diploid cell, in which respective genes are located at a homologous locus and have different mutations.

In the step of identifying at least one of a transcription product or a translation product of the second gene as the candidate target molecule for the test substance, for example, when the first gene is deleted, at least one of a transcription product or a translation product of the second gene can be identified as the candidate target molecule for the test substance.

For example, in the present invention, at least one of a gene or a protein affected by a test substance can be identified by using a knockout strain of a nonessential gene and utilizing a synthetic lethal phenotype.

For example, in some cases, a knockout strain of nonessential gene A or B can grow, while a knockout strain in which genes A and B are simultaneously disrupted result in lethality. In this case, genes A and B are in a synthetic lethal relationship. For example, when a test substance which inhibits at least one of the transcription product or the translation product of gene A is interacted with a knockout strain of gene B, it is expected that growth inhibition or lethality is caused in the gene B-knockout strain even when the concentration of the test substance is one at which the growth of a strain whose gene B is not disrupted would not be completely inhibited. By applying this principle to identify a strain in which growth inhibition or lethality is caused when a test substance is interacted with a knockout strain of a nonessential gene, a gene in a synthetic lethal relationship with the gene disrupted in this strain can be revealed. That is, a target gene of the test substance can be identified. Therefore, at least one of the transcription product or the translation product of the gene in a synthetic lethal relationship is considered to be a candidate target molecule for the test substance (Hartwell et al., Science, 1997, 278:1064-8; Parsons et al., Nat Biotechnol, 2004, 22:62-9).

Further, even when a gene in a synthetic lethal relationship with a given gene is unidentified, at least one of a target gene or a target molecule of the test substance can be presumed by a systematic analysis of the interaction of the test substance with many known knockout strains of nonessential genes. For example, a substance whose target molecule is already known may be interacted with many known knockout strains of nonessential genes, thereby obtaining a comprehensive pattern of sensitivity. This process is conducted for plural substances whose target molecules are already known, thereby obtaining a set of patterns of sensitivity. Subsequently, a test substance whose candidate target molecule has not been identified may be interacted with many knockout strains of nonessential genes to obtain a pattern of sensitivity, and the pattern may be compared with the previously-obtained patterns of sensitivity of the plural substances. As a result, when a substance showing an identical or similar pattern of sensitivity to that obtained with the test substance is found, the candidate target molecule for this substance is considered to be identical to the candidate target molecule for the test substance.

Further, for example, when strains in which a group of genes involved in a certain metabolic pathway thereof have been disrupted specifically exhibit sensitivities, at least one of a transcription product or a translation product of a gene involved in this metabolic pathway itself or involved in a pathway metabolically closely related thereto is regarded as a candidate target molecule.

Further, at least one of a gene or a protein affected by a test substance can be detected using a strain in which one of the two alleles of an essential gene is disrupted (hereinafter, sometimes referred to as a "heterozygous strain"). Here, a gene is an "essential gene" if a cell is unable to grow and dies when both of the two alleles of this gene are disrupted. The total of about 6000 genes in yeast includes about 1200 essential genes.

For example, when a test substance that inhibits at least one of a transcription product or a translation product of an essential gene, gene C, is interacted with a heterozygous strain of gene C, a function of at least one of the transcription product or the translation product of gene C may be decreased, which may result in a specific sensitivity to the test substance (Drug-induced haploinsufficiency; Giaever et al., Nature genetics, 1999, 21:278-283). By applying this principle to identify a strain that causes lethality when a test substance is interacted with a heterozygous strain, the gene disrupted in this strain can be revealed. Therefore, at least one of the transcription product or the translation product of the gene disrupted in the heterozygous strain is considered to be a candidate target molecule for the test substance.

Further, using nonessential gene knockout strains and utilizing a specific resistance to growth inhibition by a test substance, at least one of the transcription product or the translation product of a gene affected by the test substance can be detected. For example, when a test substance is a substance that exhibits toxicity only when modified by a certain protein, the toxicity is not exhibited in a strain in which the gene encoding the protein responsible for the modification is deleted. In this case, the strain in which this gene is disrupted exhibits specific resistance to the test substance.
Examples of the modification include an enzymatic chemical modification of a test substance and production of a toxic complex formed by binding of a test substance to a protein. Examples of the former modification include conversion of 5-FOA to 5-FU by the URA3 gene product. In this case, resistance to 5-FOA is provided by deletion of the URA3 gene. Examples of the latter modification include binding of rapamycin to the FPR1 protein. In this case, resistance to rapamycin is provided by deletion of the FPR1 gene (Heitman J, et al. (1991) "FK 506-binding protein proline rotamase is a target for the immunosuppressive agent FK 506 in Saccharomyces cerevisiae". Proc Natl Acad Sci U S A 88(5):1948-52).

For example, when a test substance that inhibits at least one of the transcription product or the translation product of a nonessential gene, gene D, is interacted with a knockout strain of gene D, growth of the knockout strain may not be inhibited even when the concentration of the test substance is one at which growth of the wild-type strain would be inhibited. By applying this principle to identify a strain whose growth is not inhibited when a test substance has been interacted with knockout strains of nonessential genes, the gene disrupted in this strain can be revealed. Therefore, at least one of the transcription product or the translation product of the gene disrupted in the knockout strain of nonessential gene is considered to be a candidate target molecule for the test substance or a molecule involved in the same signaling pathway as a candidate target molecule (Heitman et al., Proc Natl Acad Sci USA. 1991, 88:1948-52).

The method of analyzing a mechanism of action of a test substance with respect to yeast cells according to the present invention includes the steps of:
identifying a candidate target molecule for a test substance by the above-described method of identifying the candidate target molecule;
identifying a function of the candidate target molecule; and
analyzing a mechanism of action of the test substance with respect to yeast cells based on the function of the candidate target molecule.
By this method, efficient and precise analysis of a mechanism of action of a test substance with respect to yeast cells can be conducted.

The step of identifying a candidate target molecule for the test substance may be explained by the description of the corresponding steps of the above-described method of identifying a candidate target molecule for the test substance.
Examples of the method of identifying a function of the candidate target molecule include a method in which homologies between the sequences of a gene encoding a candidate target molecule and a gene whose function is known are compared.

Further, based on the function of the identified target molecule, a mechanism of action of the test substance with respect to yeast cells can be analyzed. For example, when the function of the target molecule is an inhibiting effect on a specific enzyme, the mechanism of action of the test substance with respect to yeast cells can be presumed to be inhibition of the enzyme.

The first screening method of the present invention is a method of screening of a substance that affects a function of a molecule of interest, and characteristically includes the steps of:
contacting at least one type of yeast cell included in the above-described set of yeast cells with a test substance;
selecting a yeast cell whose phenotype was altered by contacting with the test substance;
identifying a gene mutated in the selected yeast cell as a first gene; and
selecting a test substance, where the first gene corresponds to a gene of the target molecule.
By this method, more efficient and precise screening of a substance that specifically interacts with a gene product of a candidate target molecule can be conducted.

Each of the second and third screening methods of the present invention is a method of screening a substance that affects a function of a molecule of interest, and characteristically includes the steps of:
contacting at least one type of yeast cells included in the above-described set of yeast cells with a test substance;
selecting a yeast cell whose phenotype was altered by contacting with the test substance;
identifying a gene deleted or mutated in the selected yeast cell as a first gene;
identifying a gene which is allelic to or a gene which is in a synthetic lethal relationship with the first gene as a second gene; and
selecting a test substance, where that the second gene corresponds to a gene of the target molecule.
By this method, more efficient and precise screening of a substance that specifically interacts with a gene product of a candidate target molecule can be conducted.

Examples of the substance that affects a function of the molecule of interest herein include naturally-occurring low-molecular-weight compounds, naturally-occurring high-molecular-weight compounds, synthetic low-molecular-weight compounds, synthetic high-molecular-weight compounds, peptides, proteins, polysaccharides and nucleic acids.

According to the screening method of the present invention, a gene of a molecule that affects a function of the molecule of interest can be identified in the same manner as in the above-mentioned methods of identifying a candidate target molecule. Further, by selecting a test substance, where the identified gene corresponds to the gene of the candidate target molecule of the screening, a substance that affects a function of a molecule of interest can be screened from test substances.

### EXAMPLES

Hereinafter, the present invention will be described specifically with reference to Examples, but the present invention is not limited to these Examples.

### (Example 1)

### <Preparation of Partner Strain>

### (1) Preparation of partner strain in which PDR1 gene and PDR3 gene are disrupted

By using a method described in the method for efficient preparation of gene knockout alleles by PCR (Baudin A et.al., Nucl.Acid Res., 1993, 21, 3329-3330), a partner strain in which the PDR1 gene and the PDR3 gene are disrupted was prepared as follows.
The PDR3 gene was disrupted by replacing the PDR3 locus of the BY4741 strain (MATa) with the LEU2 gene (hereinafter referred to as KE383). Further, the PDR1 gene was disrupted by replacing the PDR1 locus of the BY4742 strain (MATα) with the HIS3 gene (hereinafter referred to as KE384). The KE383 strain and KE384 strain were mated to allow spore formation, thereby preparing a MATa Δ*met*15 strain in which both PDR1 and PDR3 are disrupted (hereinafter referred to as KE445).

### (2) Introduction of the MFA1 promoter-inducible URA3 gene to CAN1 locus

First, using the genomic DNA of a budding yeast, *S*. *cerevisiae* S288C (wild-type strain) as the template, each of: (A) a gene fragment including about 450 bp of the 5' untranslated region adjacent to the CAN1 gene; (B) a gene fragment including about 400 bp of the promoter region of the MFA1 gene; (C) a gene fragment of about 800 bp including the full-length translated region of the URA3 gene; and (D) a gene fragment including about 300 bp of the 3' untranslated region adjacent to the CAN1 gene; was amplified by PCR. Subsequently, ligation and amplification of fragments (A) and (B) by PCR was performed, and ligation and amplification of fragments (C) and (D) by PCR was performed, thereby obtaining fragment (E) and fragment (F), respectively. Finally, ligation and amplification by PCR was performed using fragments (E) and (F), thereby obtaining a 2,000-bp fragment (G) including a MFA1-URA3 marker. This fragment (G) was used to construct a plasmid. This plasmid was introduced into the KE445 strain, thereby obtaining a yeast cell line in which the CAN1 gene is replaced by fragment (G).
The sequences of the primers used for amplifying the above-described fragments by PCR are shown in Table 1 below.

**Table 1**

| PCR primer | Sequence | SEQ ID NO | Note |
|---|---|---|---|
| (A) forward | TAGGGCGAACTTGAAGAATAACC | SEQ ID NO: 1 | - |
| (A) reverse | GCCACGTTGCACACTATCCTGTGCTATGCCTTTTTTTTTTTTTGTT | SEQ ID NO: 2 | - |
| (B) forward | CAGGATAGTGTGCAACGTGGC | SEQ ID NO: 3 | - |
| (B) reverse | CTTATATGTAGCTTTCGACATTTCTATTCGATGGCTTTG | SEQ ID NO: 4 | - |
| (C) forward | ATGTCGAAAGCTACATATAAG | SEQ ID NO: 5 | - |
| (C) reverse | ATCAAAGGTAATAAAACGTCATATTTAGTTTTGCTGGCCGCATCT | SEQ ID NO: 6 | - |
| (D) forward | ATATGACGTTTTATTACCTTTGAT | SEQ ID NO: 7 | - |
| (D) reverse | ACGAAAAATGAGTAAAAATTATCTT | SEQ ID NO: 8 | - |
| (E) forward | TAGGGCGAACTTGAAGAATAACC | SEQ ID NO: 1 | (A) forward |
| (E) reverse | CTTATATGTAGCTTTCGACATTTCTATTCGATGGCTTTG | SEQ ID NO: 4 | (B) reverse |
| (F) forward | ATGTCGAAAGCTACATATAAG | SEQ ID NO: 5 | (C) forward |
| (F) reverse | ACGAAAAATGAGTAAAAATTATCTT | SEQ ID NO: 8 | (D) reverse |
| (G) forward | TAGGGCGAACTTGAAGAATAACC | SEQ ID NO: 1 | (A) forward |
| (G) reverse | ACGAAAAATGAGTAAAAATTATCTT | SEQ ID NO: 8 | (D) reverse |

The MFA1-URA3 marker function was examined in the yeast cell line constructed as described above, and it was confirmed that the yeast cell line is auxotrophic to uracil when the mating type is MATα and the uracil auxotrophy disappears when the mating type is MATa. This MATα strain was used as the partner strain (hereinafter referred to as KE458) for preparation of barrier-free YKO.

### <MATa-type barrier-free YKO>

Using MULTIMEK (384-channel automated pipettor; manufactured by Beckman Coulter), 60 µl each of a 10-fold diluted partner strain (prepared by addition of 10 ml of YPAD to KE458 in a 50 ml tube, followed by static culture for 3 to 7 days) was inoculated into a 384-well plate. Five µl each of the YKO strains (manufactured by Invitrogen, 95401.H2), which were cultured in a 384-well plate (1 strain/well), was added thereto using MULTIMEK, and then cultured at 25°C for 2 days. This plate was centrifuged at 3,000 rpm for 2 minutes, and the supernatant was discarded using MULTIMEK. The resultant was suspended by adding 20 µl of sterile water. Five µl of the suspension was diluted by mixing with 60 µl of sterile water. Five µl of the diluted suspension was added to 60 µl of a diploid selection medium (SD-(Ura+, Met+)/G418), and cultured at 30°C for 1 to 3 days to grow only diploid cells. The plate was centrifuged (3,000 rpm, for 2 minutes), and the supernatant was discarded using MULTIMEK. The resultant was suspended by adding 20 µl of sterile water. Ten µl of this suspension was spotted on Spo-agar medium using a dispenser robot SHOTMASTER 3000 (manufactured by Musashi Engineering, Inc., 96 spots/plate), and then cultured at 25°C for about 1 week to form spores.

The spores spotted on the Spo-agar medium were picked up with a disposable 96-pin replicator (Cat # 473245, manufactured by Nunc) and suspended in 20 µl of sterile water in a 96-well round-bottom plate. Ten µl of this suspension was spotted onto MATa selection agar medium (SD-(Met+)/G418/canavanine), and cultured at 30°C for 7 to 10 days to grow haploid cell colonies having the MATa mating type gene. Grown cells were picked up with a disposable 96-pin replicator, suspended in 100 µl of MATa selection liquid medium, and cultured at 25°C for 2 to 7 days. Subsequently, the cells were transferred onto a 384-well plate using a dispenser robot SHOTMASTER 3000.

Cycloheximide sensitivity was investigated on the strains obtained as described above, revealing the existence of strains that can grow in the presence of cycloheximide at a concentration of 50 ng/ml, at which concentration barrier-free yeast cannot grow. The genomic DNAs of these strains were checked by PCR, and it was revealed that these strains were MATa/MATa diploid and had at least one of the wild-type PDR1 gene or the wild-type PDR3 gene. To eliminate these diploid cells, cycloheximide sensitivity of all the strains was examined, and strains that could not grow in the presence of 50 ng/ml of cycloheximide were selected.

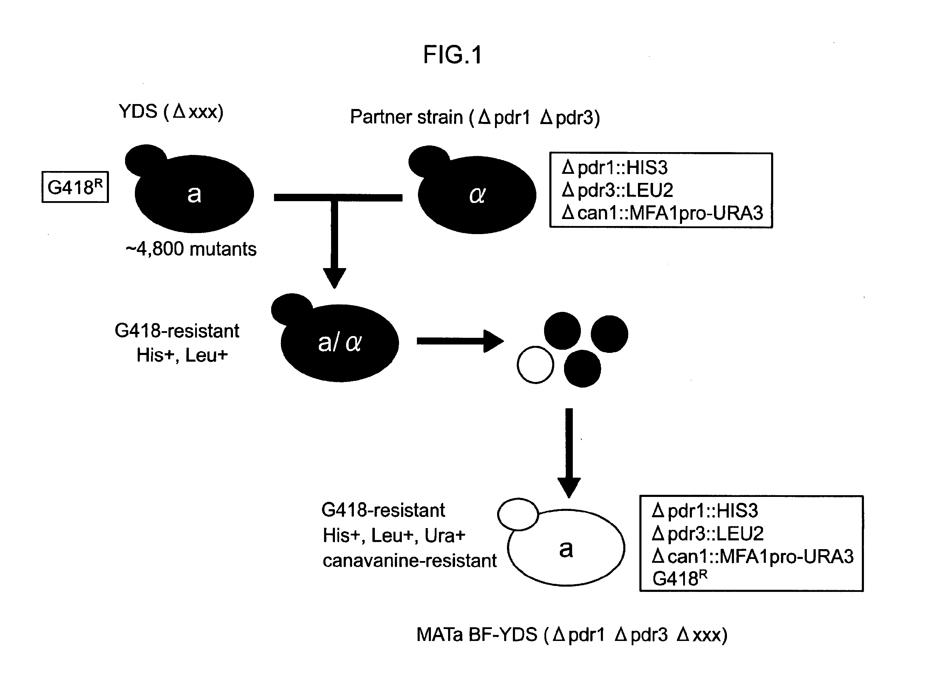

In this way, a set of yeast gene knockout strains (MATa-type barrier-free YKO) composed of 3,111 strains of yeast cells, which had MATa gene and in which the PDR1 gene and the PDR3 were disrupted, was successfully prepared (Fig. 1).

### (Example 2)

### <Preparation of MATα-type barrier-free YKO>

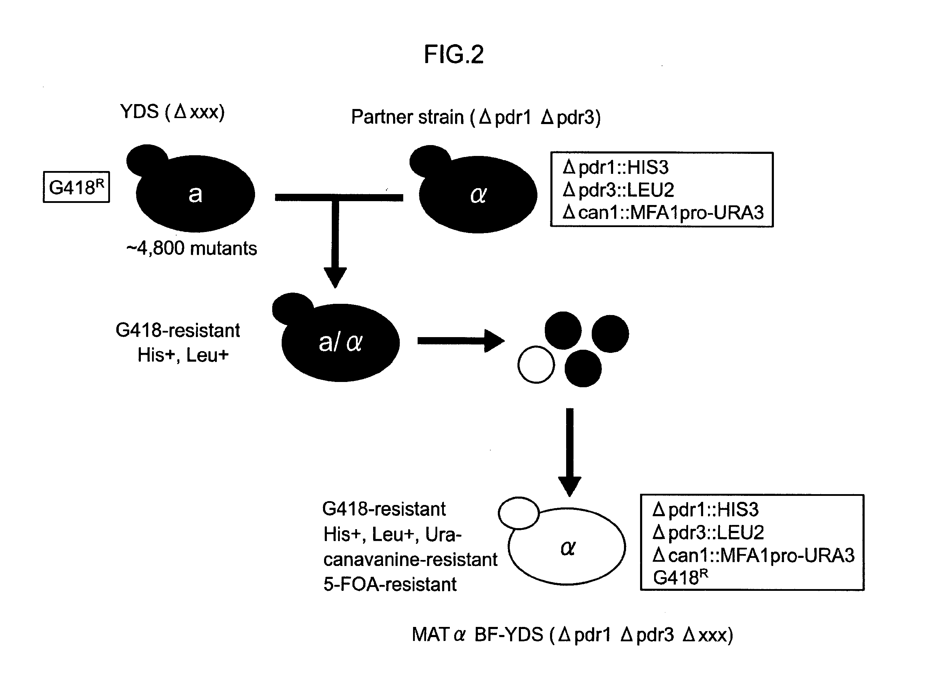

A set of yeast gene knockout strains having the MATα gene was prepared in the same manner as in Example 2 except that counter selection using 5-FOA (5-fluoroorotic acid) was conducted in place of selection of yeast cells having the MATa gene conducted in Example 1. Specifically, MATα selection agar medium (SD-(Met+, Ura+)/G418/canavanine/5FOA) was used in place of using MATa selection agar medium (SD-(Met+)/G418/canavanine), whereby a set of yeast gene knockout strains (MATα-type barrier-free YKO) composed of 1,374 strains of yeast cells, which had the MATα gene and in which the PDR1 gene and the PDR3 gene were disrupted, was successfully prepared (Fig. 2).

### (Example 3)

### <Preparation of partner strain>

### (1) Introduction of ERG6 gene regulated by GAL10 promoter

In order to prepare a partner strain in which a GAL 10 promoter is inserted in the regulatory region of the ERG6 gene, the following gene fragment was prepared.
First, primers (SEQ ID NO: 9, SEQ ID NO: 10) for the region between the GAL10 gene and the adjacent GAL1 gene were designed, and PCR was performed therewith using the yeast genomic DNA as the template to amplify the GAL 10 promoter. As the marker gene for introduction of the gene, the L-azetidine-2-carboxylic acid resistance gene of fission yeast, PPR1+, was linked to the downstream of the GAL10 promoter. Further, primers (SEQ ID NO: 11, SEQ ID NO: 12) for a region at the 5' side of the ORF of ERG6 were designed, and PCR was performed therewith using the yeast genomic DNA as the template to amplify a fragment. The obtained fragment was then linked inversely to the upstream of the GAL10 promoter. Further, primers (SEQ ID NO: 13, SEQ ID NO: 14) for the ERG6 promoter region including YML007C-A were also designed, and a fragment amplified by PCR using these primes was linked inversely to the 3' end of the ppr1+ gene. This fragment was introduced into the above-described barrier-free partner strain KE458, thereby obtaining a strain in which the fragment was correctly inserted into the 5' untranslated region of the ERG6 gene on the chromosome. In this way, a strain in which the ERG6 gene is regulated by the GAL1 promoter was prepared. This strain was backcrossed with the parent strain (BY4741), thereby obtaining a partner strain for preparation of super barrier-free YKO (hereinafter referred to as KE513).
The PCR primers used for construction of the KE513 strain are shown in Table 2 below.

**Table 2**

| SEQ ID NO | Sequence |
|---|---|
| SEQ ID NO: 9 | GCTCTAGAGCTATAGTTTTTTCTCCTTGAC |
| SEQ ID NO: 10 | AAAGCGGCCGCTTATATTGAATTTTCAAAAATT |
| SEQ ID NO: 11 | GCCCTCGAGATTGTTTAGACCGATGACGT |
| SEQ ID NO: 12 | AAACTGCAGGCAGCATAAGATGAGTGAAA |
| SEQ ID NO: 13 | GCCGAGCTCAAACAGATAAGGGAAACTTG |
| SEQ ID NO: 14 | GCCGAGCTCTTTCCGGTTCCCATGACAAA |

### <Preparation of MATa-type super barrier-free YKO>

Using MULTIMEK, 60 µl each of a 10-fold diluted partner strain (prepared by addition of 10 ml of YPAD to KE513 in a 50 ml tube, followed by static culture for 3 to 7 days) was inoculated into a 384-well plate. Five µl each of the YKO strains (manufactured by Invitrogen, 95401.H2), which were cultured in a 384-well plate (1 strain/well), was added thereto using MULTIMEK, and then cultured at 25°C for 2 days. This plate was centrifuged at 3,000 rpm for 2 minutes, and the supernatant was discarded using MULTIMEK. The resultant was suspended by adding a 20 µl of sterile water. Five µl of the suspension was diluted by mixing with 60 µl of sterile water. 5 µl of the diluted suspension was added to 60 µl of diploid selection medium (SD-(Ura+, Met+)/G418), and cultured at 30°C for 1 to 3 days to grow only diploid cells. The plate was centrifuged (3,000 rpm, for 2 minutes), and the supernatant was discarded using MULTIMEK. The resultant was suspended by adding 20 µl of sterile water. 10 µl of this suspension was spotted onto Spo-agar medium (agar medium for spore formation) using a dispenser robot SHOTMASTER 3000 (manufactured by Musashi Engineering, Inc., 96 spots/plate), and then cultured at 25°C for about 1 week to form spores.

The spores spotted onto the Spo-agar medium were picked up with a disposable 96-pin replicator (Cat # 473245, manufactured by Nunc) and suspended in 20 µl of sterile water in a 96-well round-bottom plate. Ten µl of this suspension was spotted onto MATa selection agar medium (SG-(Met+)/G418/canavanine/AZC), and cultured at 30°C for 7 to 10 days to growth haploid cell colonies having the MATa mating type gene.

Instead of picking up the entire grown colonies with the 96-pin replicator, the grown colonies were picked up one by one (1 colony/strain) with a toothpick to transfer it onto new MATa selection agar medium, and culture at 30°C for 7 days. Grown cells were picked up with a disposable 96-pin replicator, suspended in 100 µl of MATa selection liquid medium, and cultured at 25°C for 2 to 7 days. Subsequently, the grown cells were transferred to a 384-well plate using a dispenser robot SHOTMASTER 3000. All of these strains could not grow in the presence of 25 ng/ml of cycloheximide.

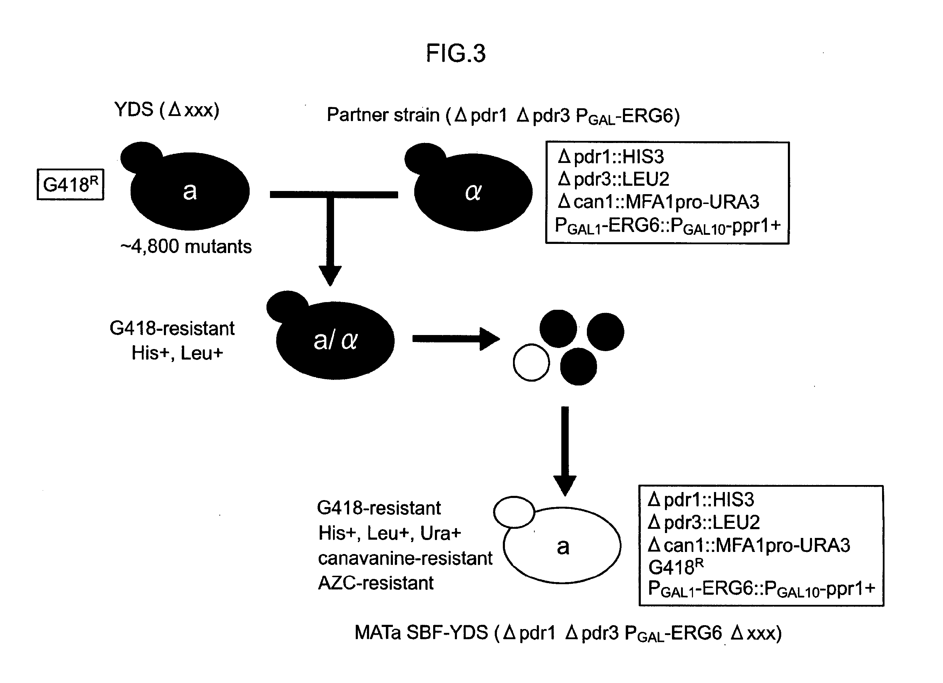

In this way, a set of yeast gene knockout strains (MATa-type super barrier-free YKO) composed of 4,044 strains of yeast cells, which had the MATa gene and in which the PDR1 gene and the PDR3 gene were disrupted and the expression of the ERG6 gene was regulated by a GAL10 promoter, was successfully prepared (Fig. 3).

Further, it can be seen that, by conducting a process for selecting single colonies, contamination with diploid yeast cells, which originally exist at a low frequency but grow quickly, can be effectively prevented.

### (Example 4)

### <Preparation of diploid barrier-free YKO>

By mating the MATa-type barrier-free YKO yeast prepared in Example 1 with the MATα-type barrier-free YKO yeast, in which corresponding nonessential gene is disrupted, prepared in Example 2, a set of diploid yeast gene knockout strains whose nonessential gene was disrupted (diploid barrier-free YKO) was prepared.

### (Example 5)

### <Evaluation of sensitivity to lovastatin>

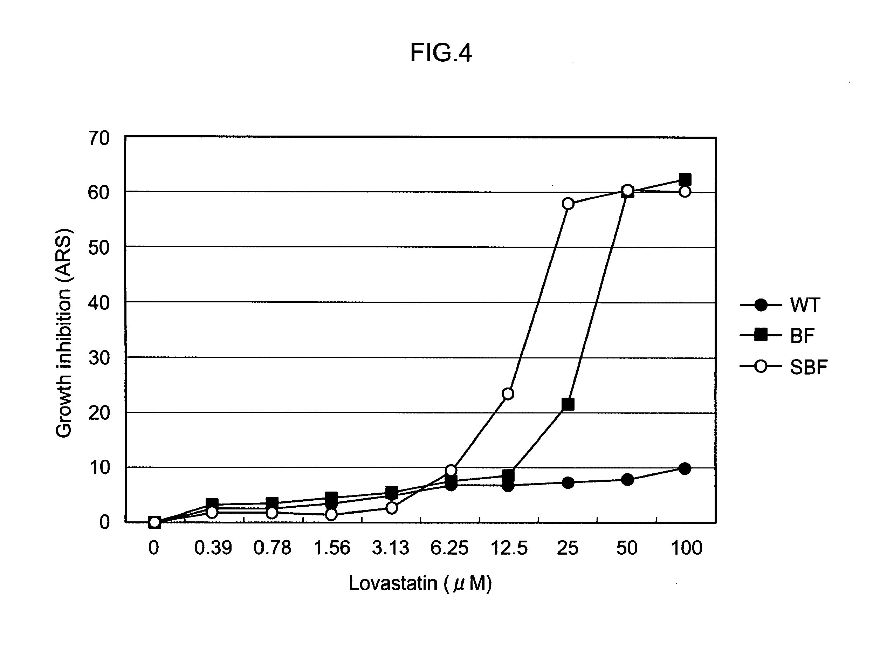

To confirm drug sensitivity of the barrier-free YKO (BF-YKO) and super barrier-free YKO (SBF-YKO) prepared as above, changes in sensitivity of the yeasts caused by proliferation inhibiting effect of lovastatin were examined using a parent strain and a partner strain.
A lovastatin solution was added to a 96-well plate such that the concentration of lovastatin (Sigma-Aldrich, #M2147) was within the range of from 0.39 µM to 100 µM. A parent strain (WT:BY4742, Open Biosystems, #YSC1049) a partner strain for barrier-free YKO (BF:KE458), and a partner strain for super barrier-free YKO (SBF:KE513), which had been subjected to static culture at room temperature for 2 days, were added to the plate such that the final cell number was at a 2000-fold dilution, and then subjected to static culture at 30°C.
Thereafter, light absorbance thereof at 620 nm was measured over time using a plate reader (SUNRISE, manufactured by Tecan) for 48 hours. Calculation of the cell proliferation inhibiting activity was carried out using the area score method (ARS) described in Japanese Patent Application Laid-Open No.2006-141298. In ARS, a larger value indicates a greater cell proliferation inhibiting activity. The results are shown in Fig. 4.
Fig. 4 shows that only a slight cell proliferation inhibiting effect was observed in the parent strain (WT) even when the lovastatin concentration was 100 µM; in contrast, a BF strain exhibited maximum inhibiting activity at a concentration of 50 µM and an SBF strain exhibited maximum inhibiting activity at a concentration of 25 µM.
Further, it can be seen that sensitizations of the BF and SBF strains were 8 and 16 times higher, respectively, than the WT strain, when compared based on the lovastatin concentrations at which an area score of about 10 was obtained.

### (Example 6)

### <Identification of target molecule of lovastatin and analysis of mechanism of action using super barrier-free YKO>

Identification of a target molecule of lovastatin was conducted using the super barrier-free YKO (SBF-YKO) composed of 4,044 strains prepared as above. The SBF-YKO strains were inoculated into twelve 384-well plates (1 strain/well) in an ordered manner, and subjected to 3 to 7 days of static culture to equalize the number of cells across all strains. The cells were diluted 196-fold, thereby obtaining a cell dilution.
On the other hand, 384-well plates in which 20 µl of YPAD medium containing 3.9 µM lovastatin was added to each well, and 384-well plates in which 20 µl of lovastatin-free YPAD medium was added to each well were prepared. 5 µl of the obtained cell dilution was added to each well. By this operation, the cells were diluted 1000-fold and the compound concentration was adjusted to 3.125 µM.
These cells were cultured at 25°C, and light absorbance thereof at 650 nm was measured over time using a plate reader (SPECTRAMAX340PC384, Molecular Devices) from about 22 hours to 48 hours after the beginning of the culture. The area scores (ARS) were calculated based on the obtained data, thereby ranking the sensitivity.

As a result, a knockout strain of the HMG1 gene encoding HMG-CoA reductase, which is a target molecule of lovastatin, was found as a strain exhibiting high sensitivity. Since *S*. *cerevisiae* also has the HMG2 gene encoding the same reductase as HMG1, disruption of HMG1 is not lethal to yeast. However, since the amount of the enzyme produced by HMG2 is about one fifth of that produced by HMG1, it can be easily understood that the lovastatin concentration at which the enzyme remaining in a HMG1-knockout strain is inhibited is lower than that of a strain having the HMG1 gene.
Further, a BTS1-knockout strain was found as the most sensitive knockout strain. BTS1 encodes famesyltransferase and acts downstream of HMG1 in the sterol biosynthetic pathway. In the BTS1-knockout strain, it is thought that downstream events are interrupted, which results in a stronger inhibiting effect of HMG-CoA reductase compared to other strains.

### (Example 7)

### Identification of target molecule of lovastatin and analysis of mechanism of action using barrier-free YKO

Using the barrier-free YKO (BF-YKO) composed of 3,011 strains as prepared above, a target molecule of lovastatin was identified. The strains of BF-YKO were inoculated into twelve 384-well plates (1 strain/well) in an ordered manner. The static culture was performed for 3 to 7 days to equalize the number of cells across all strains. The cells were diluted 196-fold, thereby obtaining a cell dilution.
On the other hand, 384-well plates wherein 20 µl of YPAD medium containing 3.9 µM, 7.8 µM or 15.6 µM lovastatin was added to each well, and 384-well plates in which 20 µl of lovastatin-free YPAD medium was added to each well were prepared. 5 µl of the obtained cell dilution was added to each well. By this operation, the cells were diluted 1000-fold and the respective concentrations of the compound were adjusted to 3.125 µM, 6.25 µM and 12.5 µM.
These cells were cultured at 25°C, and light absorbance thereof at 650 nm was measured over time using a plate reader (SPECTRAMAX340PC384, Molecular Devices) from about 22 hours to 48 hours after the beginning of the culture. The area scores (ARS) were calculated based on the obtained data, thereby ranking the sensitivity.

As a result, it was found that a knockout strain of the HMG1 gene encoding HMG-CoA reductase, which is a target molecule of lovastatin, exhibits a high sensitivity in the cases of the compound concentrations of 6.25 µM and 12.5 µM. Therefore, it can be seen that a target molecule of lovastatin can be identified by using barrier-free YKO even when the compound concentration is low.

### (Comparative Example 1)

### <Identification of target molecule of lovastatin and analysis of mechanism of action using YKO collection>

Identification of a target molecule of lovastatin was conducted in the same manner as in Example 7, except that commercially available YKO (Invitrogen, #95401.H2) composed of 4,847 strains was used instead of BF-YKO.
As a result, a knockout strain of the HMG1 gene encoding HMG-CoA reductase, which is a target molecule of lovastatin, was not found as a strain exhibiting sensitivity in both cases of the compound concentrations of 3.125 µM and 12.5 µM. Similarly, a BTS1-knockout strain also was not found as a strain exhibiting sensitivity.

### (Example 8)

### <Evaluation of sensitivity to rapamycin>

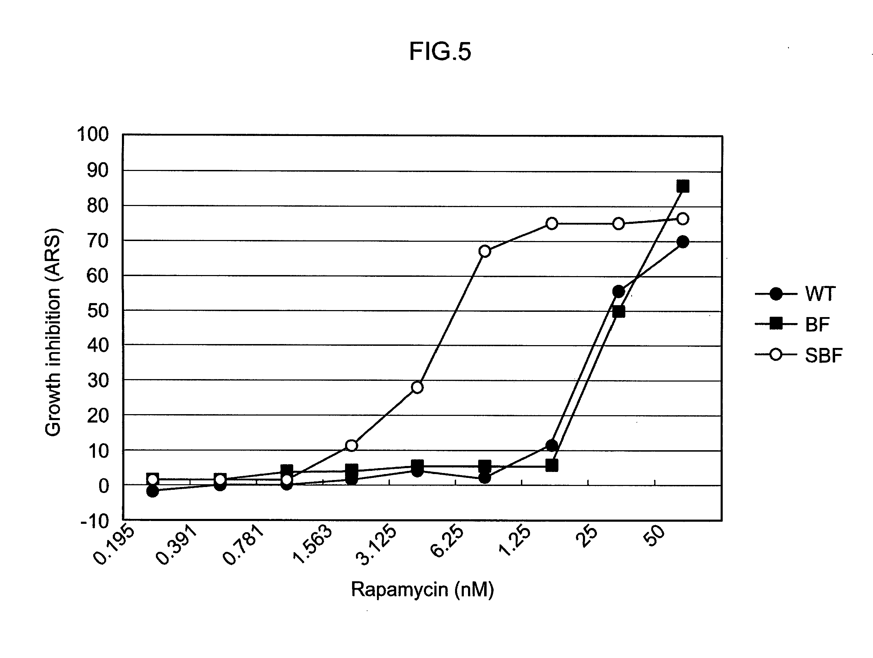

In the same manner as in Example 5, to confirm drug sensitivity of the barrier-free YKO (BF-YKO) and super barrier-free YKO (SBF-YKO), changes in sensitivity of the yeasts caused by the proliferation inhibiting effect of rapamycin were examined using a parent strain and a partner strain.
A rapamycin solution was added to a 96-well plate such that the concentration of rapamycin (Sigma-Aldrich, #R0395) was varied within the range of from 0.2 nM to 50 nM. A parent strain (WT:BY4741, Open Biosystems, #YSC1048), a barrier-free strain (BF:KE457), and a super barrier-free strain (SBF:KE477), which had been subjected to static culture at room temperature for 2 days, were added such that the final cell numbers was at a 1000-fold dilution, and then subjected to static culture at 30°C.
Thereafter, light absorbance thereof at 620 nm was measured over time using a plate reader (SUNRISE, manufactured by Tecan) for 48 hours. Calculation of the cell proliferation inhibiting activity was carried out using the area score method (ARS) described in Japanese Patent Application Laid-Open No. 2006-141298. In ARS, a larger value indicates a greater cell proliferation inhibiting activity. The results are shown in Fig. 5.

Fig. 5 shows that maximum cell proliferation inhibiting effect was observed in the parent strain (WT) and a BF strain at a rapamycin concentration of 50 nM, in contrast, an SBF strain exhibited maximum effect at a rapamycin concentration of 6.25 nM.
Further, it can be seen that sensitization of the SBF strain was 8 times higher than the WT strain, when compared based on the rapamycin concentrations at which an area score of about 10 was obtained.

### (Example 9)

### <Identification of target molecule of rapamycin and analysis of mechanism of action using super barrier-free YKO>

In the same manner as in Example 6, identification of a target molecule of rapamycin was conducted using super barrier-free YKO (SBF-YKO). Each of the SBF-YKO strains was subjected to 3 to 7 days of static culture to equalize the number of cells across all strains. The cells were diluted 196-fold, thereby obtaining a cell dilution.
On the other hand, 384-well plates in which 20 µl of YPAD medium containing 1.25 nM rapamycin was added to each well, and 384-well plates in which 20 µl of rapamycin-free YPAD medium was added to each well were prepared. Five µl of the prepared cell dilution was added to each well. By this operation, the cells were diluted 1000-fold and the concentration of the compound was adjusted to 1 nM.
As a result, a knockout strain of the TOR1 gene encoding TOR kinase, which is a target molecule of rapamycin, was found as a strain exhibiting a high sensitivity at a rapamycin concentration of 1 nM.
Since *S*. *cerevisiae* also has the TOR2 gene encoding the same enzyme as TOR1, disruption of TOR1 is not lethal to yeast. However, it can be easily understood that the rapamycin concentration, at which the enzyme remaining in a TOR1-knockout strain is inhibited, would be lower than that of a strain having the TOR1 gene.
Further, since TOR2 is essential for survival, SBF-YKO, which is a set of nonessential gene knockout strains, did not contain a TOR2-knockout strain. Therefore, a TOR2-knockout strain was not identified as a sensitive strain in the test using SBF-YKO.

### (Comparative Example 2)

### <Identification of target molecule of rapamycin and analysis of mechanism of action using YKO collection>

Identification of a target molecule of rapamycin was conducted in the same manner as in Example 9, except that commercially available YKO (Invitrogen, #95401.H2) composed of 4,847 strains was used instead of SBF-YKO and that the compound concentrations were changed to 10 nM, 12.5 nM and 15 nM.

As a result, a knockout strain of the TOR1 gene encoding TOR kinase, which is a target molecule of rapamycin, was found as a strain exhibiting a high sensitivity at the compound concentrations of 10 nM, 12.5 nM and 15 nM.

From these results, it was shown that a target molecule can be identified and the mechanism of action can be predicted by using BF-YKO or SBF-YKO, even when a low concentration of lovastatin is used. Especially, it was shown that a target molecule can be identified and the mechanism of action can be predicted by using SBF-YKO, even when the compound concentration is as low as 3.125 µM. Further, a target molecule can be identified and the mechanism of action can be predicted by using SBF-YKO, even when rapamycin is used at a concentration as low as one tenth of that of YKO.

### Industrial Applicability

The set of yeast cells according to the present invention is industrially extremely useful since it can be suitably applied to highly-sensitive identification of a candidate target molecule, analysis of an mechanism of action and screening.

## Claims

1. A set of yeast cells comprising two or more types of yeast cells, wherein at least one mutually different gene has been deleted or mutated in each of the two or more types of yeast cells, and all of the two or more types of yeast cells are drug hypersensitive yeast cells in which the expression of at least one drug sensitivity-related gene is regulated.

2. The set of yeast cells according to claim 1, wherein the at least one drug sensitivity-related gene is at least one selected from a transporter-related gene or an ergosterol biosynthesis-related gene.

3. The set of yeast cells according to claim 2, wherein the transporter-related genes are PDR1 and PDR3.

4. The set of yeast cells according to claim 2, wherein the ergosterol biosynthesis-related gene is ERG6 or ERG3.

5. The set of yeast cells according to claim 2, wherein the expression of the transporter-related gene is regulated by deletion of the transporter-related gene, and the expression of the ergosterol biosynthesis-related gene is regulated by transcriptional regulation of the ergosterol biosynthesis-related gene.

6. The set of yeast cells according to claim 1, wherein the yeast cells are diploid.

7. A method of identifying a candidate target molecule for a test substance, the method comprising the steps of:
contacting yeast cells included in the set of yeast cells according to claim 1 with a test substance;
selecting a yeast cell whose phenotype was altered by contacting with the test substance;
identifying a gene mutated in the selected yeast cell as a first gene; and
identifying at least one of a transcription product or a translation product of the first gene as a candidate target molecule for the test substance.

8. A method of identifying a candidate target molecule for a test substance, the method comprising the steps of:
contacting yeast cells included in the set of yeast cells according to claim 1 with a test substance;
selecting a yeast cell whose phenotype was altered by contacting with the test substance;
identifying a gene deleted or mutated in the selected yeast cell as a first gene;
identifying a gene allelic to the first gene as a second gene; and
identifying at least one of a transcription product or a translation product of the second gene as a candidate target molecule for the test substance.

9. A method of identifying a candidate target molecule for a test substance, the method comprising the steps of:
contacting yeast cells included in the set of yeast cells according to claim 1 with a test substance;
selecting a yeast cell whose phenotype was altered by contacting with the test substance;
identifying a gene deleted or mutated in the selected yeast cell as a first gene;
identifying a gene which is in a synthetic lethal relationship with the first gene as a second gene; and
identifying at least one of a transcription product or a translation product of the second gene as a candidate target molecule for the test substance.

10. The method of identifying a candidate target molecule according to any one of claims 7 to 9, wherein the alteration of the phenotype is lethality or growth inhibition.

11. The method of identifying a candidate target molecule according to claim 10, wherein the selecting of the yeast cells comprises the steps of:
(1) obtaining a function F(t) of a control curve of an index relating to a number of cells in the absence of a test substance over time, and a function G(t) of a test curve of an index relating to a number of cells in the presence of the test substance over time;
(2) calculating an integrated value of the formula (F(t)-G(t)) to obtain an area value; and
(3) selecting a yeast cell, whose phenotype was altered, based on the area value.

12. A method of analyzing a mechanism of action of a test substance with respect to yeast cells, the method comprising the steps of:
identifying a candidate target molecule for a test substance by the method of identifying the candidate target molecule according to any one of claims 7 to 9;
identifying a function of the candidate target molecule; and
analyzing a mechanism of action of the test substance with respect to yeast cells based on the function of the candidate target molecule.

13. A method of screening a substance that affects a function of a target molecule, the method comprising the steps of:
contacting yeast cells included in the set of yeast cells according to claim 1 with a test substance;
selecting a yeast cell whose phenotype was altered by contacting with the test substance;
identifying a gene mutated in the selected yeast cell as a first gene; and
selecting a test substance, where the first gene corresponds to a gene whose transcription product or translation product is the target molecule.

14. A method of screening a substance that affects a function of a target molecule, the method comprising the steps of:
contacting yeast cells included in the set of yeast cells according to claim 1 with a test substance;
selecting a yeast cell whose phenotype was altered by contacting with the test substance;
identifying a gene deleted or mutated in the selected yeast cell as a first gene;
identifying a gene allelic to the first gene as a second gene; and
selecting a test substance, where the second gene corresponds to a gene whose transcription product or translation product is the target molecule.

15. A method of screening a substance that affects a function of a target molecule, the method comprising the steps of:
contacting yeast cells included in the set of yeast cells according to claim 1 with a test substance;
selecting a yeast cell whose phenotype was altered by contacting with the test substance;
identifying a gene deleted or mutated in the selected yeast cell as a first gene; and
identifying a gene which is in a synthetic lethal relationship with the first gene as a second gene; and
selecting a test substance, where the second gene corresponds to a gene whose transcription product or translation product is the target molecule.

16. The method of screening according to any one of claims 13 to 15, wherein the alteration of the phenotype is lethality or growth inhibition.

17. The method of screening according to claim 16, wherein selecting the yeast cells comprises the steps of:
(1) obtaining a function F(t) of a control curve of an index relating to a number of cells in the absence of a test substance over time, and a function G(t) of a test curve of an index relating to a number of cells in the presence of the test substance over time;
(2) calculating an integrated value of the formula (F(t)-G(t)) to obtain an area value; and
(3) selecting a yeast cell, whose phenotype was altered, based on the area value.


SEQUENCE LISTING

<110>  Eisai Co., Ltd.

<120>  Yeast strain set and methods for identifying a target molecule,
       analysing an action mechanism and screening  a target moleclule

<130>  CO-F03300-00

<160>  14

<170>  PatentIn version 3.4

<210>  1
<211>  23
<212>  DNA
<213>  Artificial

<220>
<223>  PCR primer

<400>  1
tagggcgaac ttgaagaata acc                                              23


<210>  2
<211>  46
<212>  DNA
<213>  Artificial

<220>
<223>  PCR primer

<400>  2
gccacgttgc acactatcct gtgctatgcc tttttttttt tttgtt                     46


<210>  3
<211>  21
<212>  DNA
<213>  Artificial

<220>
<223>  PCR primer

<400>  3
caggatagtg tgcaacgtgg c                                                21


<210>  4
<211>  39
<212>  DNA
<213>  Artificial

<220>
<223>  PCR primer

<400>  4
cttatatgta gctttcgaca tttctattcg atggctttg                             39

<210>  5
<211>  21
<212>  DNA
<213>  Artificial

<220>
<223>  PCR primer

<400>  5
atgtcgaaag ctacatataa g                                                21


<210>  6
<211>  45
<212>  DNA
<213>  Artificial

<220>
<223>  PCR primer

<400>  6
atcaaaggta ataaaacgtc atatttagtt ttgctggccg catct                      45


<210>  7
<211>  24
<212>  DNA
<213>  Artificial

<220>
<223>  PCR primer

<400>  7
atatgacgtt ttattacctt tgat                                             24


<210>  8
<211>  25
<212>  DNA
<213>  Artificial

<220>
<223>  PCR primer

<400>  8
acgaaaaatg agtaaaaatt atctt                                            25


<210>  9
<211>  30
<212>  DNA
<213>  Artificial

<220>
<223>  PCR primer

<400>  9
gctctagagc tatagttttt tctccttgac                                       30


<210>  10

<211>  33
<212>  DNA
<213>  Artificial

<220>
<223>  PCR primer

<400>  10
aaagcggccg cttatattga attttcaaaa att                                    33


<210>  11
<211>  29
<212>  DNA
<213>  Artificial

<220>
<223>  PCR primer

<400>  11
gccctcgaga ttgtttagac cgatgacgt                                         29


<210>  12
<211>  29
<212>  DNA
<213>  Artificial

<220>
<223>  PCR primer

<400>  12
aaactgcagg cagcataaga tgagtgaaa                                         29


<210>  13
<211>  29
<212>  DNA
<213>  Artificial

<220>
<223>  PCR primer

<400>  13
gccgagctca aacagataag ggaaacttg                                         29


<210>  14
<211>  29
<212>  DNA
<213>  Artificial

<220>
<223>  PCR primer

<400>  14
gccgagctct ttccggttcc catgacaaa                                         29